# EUROPEAN PATENT APPLICATION

(11) **EP 4 029 538 A1**
(43) Date of publication of application: **20.07.2022**
(21) Application number: 20862738.0
(22) Date of filing: 08.09.2020
(51) Int. Cl.: A61L 27/30, A61L 27/32, A61L 27/54

(54) **BIOCOMPATIBLE IMPLANT AND PREPARATION METHOD OF BIOCOMPATIBLE IMPLANT**

(30) Priority: 11.09.2019 JP 2019165593
(71) Applicant: Kyocera Corporation, Kyoto-shi Kyoto 612-8501 (JP)
(72) Inventor: MURAKAMI, Takayuki, Kyoto-shi, Kyoto 612-8501 (JP); NAKAMURA, Taito, Kyoto-shi, Kyoto 612-8501 (JP)
(74) Representative: TBK
(86) International application number: PCT/JP2020/033973
(87) International publication number: WO 2021/049494

(57) **Abstract**

A biocompatible implant according to one aspect of the present disclosure includes a base and a calcium phosphate coating film located on a surface of the base and including silver. The calcium phosphate coating film includes a plurality of calcium phosphate particles located on a surface thereof, and a plurality of needle-like crystals located on a surface of the plurality of calcium phosphate and the plurality of needle-like crystals are located in a gap at an interface between adjacent calcium phosphate particles among the plurality of calcium phosphate particles.

## Description

### Cross-reference to related applications

This application claims the priority of JP 2019-165593 filed on September 11, 2019, and the entire disclosure of the earlier application is incorporated herein by reference.

### Technical Field

The present disclosure relates to a biocompatible implant.

### Background Art

A biocompatible implant provided with a coating film expressing antimicrobial properties is known.

### Summary

A biocompatible implant according to one aspect of the present disclosure includes a base and a calcium phosphate coating film located on a surface of the base and including silver. The calcium phosphate coating film includes a plurality of calcium phosphate particles located on a surface thereof, and a plurality of needle-like crystals located on a surface of the plurality of calcium phosphate particles, and the plurality of needle-like crystals is located in a gap at an interface between adjacent calcium phosphate particles among the plurality of calcium phosphate particles.

### Brief Description of Drawings

FIG. 1 is a schematic view illustrating a biocompatible implant of an embodiment.
FIG. 2 is a scanning electron microscope (SEM) photograph of a surface of a specimen of Example 1.
FIG. 3 is a SEM photograph of a cross section near a surface including a coating film of a specimen of Example 2.
FIG. 4 is a SEM photograph of a surface of a specimen of Comparative Example 1.

### Description of Embodiments

In a conventional coating film, since decomposition and absorption in a living body are fast, there is a concern that a period until the coating film disappears is as short as several months to several years. When the coating film disappears, antimicrobial function is also lost, and thus there is a risk that antimicrobial properties cannot be maintained over a long period of time, such as longer than several years. According to one aspect of the present disclosure, the antimicrobial properties can be maintained over a long period of time.

### Biocompatible Implant

The following describes in detail a biocompatible implant 1 of an embodiment while referencing the drawings. However, the figures referred to below are simplified representations of only a configuration necessary for description of the embodiment. Thus, the biocompatible implant 1 may include any configuration not illustrated in the figures referred to. Further, the dimensions of the configuration in the figures do not faithfully represent the actual dimensions of the configuration, the dimension ratios of the configuration, or the like.

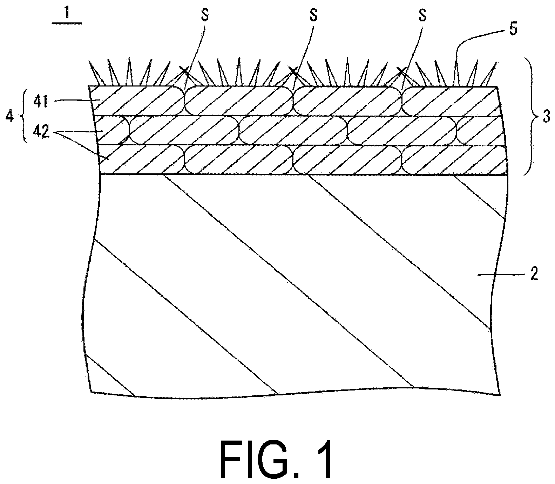

As illustrated in FIG. 1, the biocompatible implant 1 includes a base 2 and a calcium phosphate coating film 3 (hereinafter, also referred to as "coating film 3") located on a surface of the base 2 and including silver.

Examples of a material of the base 2 include a metal, a ceramics, and a plastic. Examples of the metal include stainless steel alloys, cobalt chromium alloys, titanium, titanium alloys, alumina, and zirconia. Examples of titanium alloys include alloys to which at least one of, for example, aluminum, tin, zirconium, molybdenum, nickel, palladium, tantalum, niobium, vanadium, and platinum is added. Specific examples of titanium alloys include Ti-6A1-4V alloy. Examples of the ceramics include, for example, alumina, zirconia, and an alumina-zirconia composite ceramics. Examples of the plastic include, for example, polyethylene, fluorine-based resin, epoxy resin, PEEK resin, and Bakelite.

The coating film 3 contains silver having an antimicrobial function. The silver in the coating film 3 is, in the present embodiment, metal silver or a silver compound, and is dispersed and distributed in the coating film 3 as irregularly shaped particles each being 1 nm or greater. Note that the silver in the coating film 3 may be included in the form of silver ions in the calcium phosphate based material forming the coating film 3. Both silver in the form of particles and silver in the ionic state may also be included. The upper limit of the size of each of the irregularly shaped particles may be 10 µm or less. The content of silver in the coating film 3 may be, for example, from 0.05 to 10 mass%. The thickness of the coating film 3 may be, for example, from 5 to 300 µm. The coating film 3 may be a single layer or multiple layers.

The coating film 3 is formed of a calcium phosphate based material. Examples of the calcium phosphate based material include hydroxyapatite (hereafter, also referred to as "HA"), α-tricalcium phosphate, β-tricalcium phosphate or tetracalcium phosphate. The exemplified calcium phosphate based materials may be used in a single or a mixture of two or more types.

The coating film 3 may be located on a portion of a surface of the base 2, or may be located on the entire surface of the base 2. Examples of a method for disposing the coating film 3 on the surface of the base 2 include, for example, a thermal spraying method, a vapor deposition method, and a wet coating method. Examples of the thermal spraying method include, for example, a flame thermal spraying method, a high-speed flame thermal spraying method, a plasma thermal spraying method, and a cold spraying method. Examples of the vapor deposition method include, for example, a physical vapor deposition method such as a sputtering method, an ion plating method, an ion beam deposition method, or an ion mixing method. Examples of the wet coating method include, for example, a sol-gel method.

The coating film 3 may be a calcium phosphate thermally sprayed coating film (hereinafter, also referred to as "thermally sprayed coating film"). The thermally sprayed coating film is a coating film disposed on the surface of the base 2 by the thermal spraying method. Specific examples of the thermally sprayed coating film include a silver hydroxyapatite thermally sprayed coating film. The silver HA thermally sprayed coating film is a thermally sprayed coating film including silver and HA.

The coating film 3 includes a plurality of calcium phosphate particles 4 (hereinafter, also referred to as "particles 4") located on the surface of the coating film 3 and a plurality of needle-like crystals 5 located on the surface of the plurality of particles 4. The plurality of needle-like crystals 5 is located in a gap S at an interface between adjacent ones of the particles 4 among the plurality of particles 4. According to this configuration, a state in which the coating film 3 contains silver is easily maintained. Thus, antimicrobial properties can be maintained over a long period of time. The above configuration may be, for example, magnified and evaluated using a scanning electron microscope (hereinafter, also referred to as "SEM").

Here, the degree of crystallinity of the coating film 3 may be greater than 90%. Such a degree of crystallinity is greater than the degree of crystallinity of a conventional coating film. In a coating film 3 having a degree of crystallinity of greater than 90%, decomposition and absorption (dissolution rate) in a living body is slower than that of a conventional coating film, and accordingly, the amount of elution of silver ions is less than that of a conventional coating film. Thus, in a case where the coating film 3 contains silver in the same concentration as the conventional coating film, the initial antimicrobial properties are lower than those of the conventional coating film, but since the rate of dissolution in a living body is relatively slow, the period until the coating film 3 disappears is longer than that of the conventional coating film, and as a result, the period during which silver ions can be eluted in a living body is longer than that of the conventional coating film. Thus, the antimicrobial properties can be maintained over a long period of time. The antimicrobial properties can also be expected to be maintained over a long period of time beyond years.

The upper limit value of the degree of crystallinity of the coating film 3 may be 100% or less. The degree of crystallinity of the coating film 3 may be measured using an X-ray diffraction apparatus (hereafter, also referred to as "XRD") based on ISO13779-3.

The particles 4 are formed of the calcium phosphate based material. The particles 4 are not limited to a particular size. For example, the particle size of each of the particles 4 may be from 0.5 to 100 µm. The particle size may be measured using SEM.

As in an example illustrated in FIG. 1, the particles 4 may be flat thermally sprayed particles (molten particles) formed when the coating film 3 is disposed on the surface of the base 2 by the thermal spraying method. In this case, in addition to the surface of the coating film 3, the particles 4 are located inside the coating film 3 as weld particles. The particles 4 located on the surface of the coating film 3 may be calcium phosphate first particles 41 (hereinafter, also referred to as "first particles 41"), and the particles 4 located closer to the base 2 side than the first particles 41 may be calcium phosphate second particles 42 (hereinafter, also referred to as "second particles 42"). Note that the particles 4 need not contain silver. The silver may be located inside the particles 4 as irregularly shaped particles and may be located in the gap S at an interface between adjacent ones of the first particles 41. The silver may also be located in a gap at an interface between adjacent ones of the first particles 41 and the second particles 42 and in a gap at an interface between adjacent ones of the second particles 42.

The plurality of needle-like crystals 5 may be located on a portion of the surface of the particles 4 or may be located on the entire surface of the particles 4. In a case where the plurality of needle-like crystals 5 are located on the entire surface of the particles 4, it is easy for the coating film 3 to maintain its silver content.

The needle-like crystals 5 may be formed of the calcium phosphate based material. The needle-like crystals 5 need not contain silver. The needle-like crystals 5 may be precipitated from the particles 4.

The needle-like crystals 5 may be smaller than the particles 4. Note that each of the needle-like crystals 5 is not limited to a specific size. For example, a major axis of each of the needle-like crystals 5 may be from 0.1 to 50 µm. A minor axis of each of the needle-like crystals 5 may be from 0.001 to 10 µm. The sizes of the major axis and the minor axis may be measured using SEM.

The coating film 3 may include dendritic crystals located therein. In this case, since the coating film 3 is densified, the period until the coating film 3 disappears is long. Thus, the antimicrobial properties can be maintained over a long period of time. Note that the inside of the coating film 3 may be formed of the dendritic crystals. In a case where the coating film 3 includes the particles 4, the dendritic crystals may be located inside the particles 4. The dendritic crystals may also be referred to as dendrites. The above configuration may be, for example, evaluated by magnifying and observing the cross section of the coating film using SEM.

The biocompatible implant 1 includes a biocompatible implant, such as an artificial bone or an inner fixing tool used for treatment of diseases or injuries, a prosthetic joint used for restoring lost joint functions, a dental implant used for restoring teeth, and a spinal implant used for treating diseases of vertebral bodies and intervertebral discs. Note that the biocompatible implant 1 is not limited to the illustrated examples.

### Method of Manufacturing Biocompatible Implant

Next, a description will be given for the method of manufacturing the biocompatible implant according to the embodiment with reference to a case of manufacture of the biocompatible implant 1 using the flame thermal spraying method.

In the flame thermal spraying method, a gas flame of a combustible gas and oxygen is used as the heat source. Then, a powder of the calcium phosphate based material to which silver oxide is added is used as the thermal spraying material, the thermal spraying material is brought into a state of being molten or being nearly molten, and the thermal spraying material is sprayed onto the surface of the base 2 to form the coating film 3 (thermally sprayed coating film), thereby obtaining the biocompatible implant 1.

The thermal spraying temperature may be about 2700°C. The thermal spraying speed may be Mach 0.6. The flame thermal spraying may be performed by, for example, introducing the thermal spraying material using dry air of 100 psi into a gas flame torch of oxygen gas 50 psi and acetylene gas 43 psi, under the conditions of setting the thermal spraying distance to be from 60 to 100 mm.

The formed coating film 3 may be subjected to a hydrothermal treatment. In this case, the degree of crystallinity of the coating film 3 easily exceeds 90%. The needle-like crystals 5 are easily precipitated from the particles 4. A plurality of the needle-like crystals 5 are likely to be located in the gap S. The plurality of dendritic crystals are also likely to be located inside the coating film 3.

The hydrothermal treatment is a treatment that involves heating to a temperature above 100°C in a pressure vessel containing water. Examples of the pressure vessel include a high-pressure cylinder. The conditions of the hydrothermal treatment may be, for example, set as follows.
Liquid volume: from 5 to 7 ml/cm²
Temperature: from 130 to 155°C
Time: from 11 to 25 hours

The present disclosure will be described in detail below using examples, but the present disclosure is not limited to the following examples.

### Examples

### Example 1

### Fabrication of Specimens

First, a 50 mm × 50 mm × 2 mm titanium plate was prepared. Next, HA powder containing 3 mass% of silver oxide and 97 mass% of HA was used as the thermal spraying material, and the thermal spraying material was sprayed by the flame thermal spraying method on one surface of the titanium plate, thereby forming a silver HA thermally sprayed coating film having a thickness of 30 µm. Finally, the silver HA thermally sprayed coating film was subjected to hydrothermal treatment, thereby obtaining specimens.

The flame thermal spraying was performed under the conditions of introducing the thermal spraying material using dry air of 100 psi into a gas flame torch of oxygen gas 50 psi and acetylene gas 43 psi, and setting the thermal spraying distance to be from 60 to 100 mm.

The hydrothermal treatment was performed under the following conditions in a high pressure cylinder.
Liquid volume: 6 ml/cm²
Temperature: 135°C
Time: 24 hours

The degree of crystallinity of the obtained specimens was measured using XRD based on ISO13779-3. The result is described below.
Degree of crystallinity: 91%

### Example 2

Specimens were fabricated under the same conditions as in Example 1 with the exception that the hydrothermal treatment was performed under the conditions indicated below.
Liquid volume: 6 ml/cm²
Temperature: 150°C
Time: 24 hours

The degree of crystallinity of the obtained specimens was measured in the same manner as in Example 1. The result is described below.
Degree of crystallinity: 100%

### Comparative Example 1

Specimens were fabricated under the same conditions as in Example 1 with the exception that a vacuum thermal treatment instead of the hydrothermal treatment was applied to the silver HA thermally sprayed coating film. The vacuum thermal treatment was performed in a vacuum oven under the following conditions.
Temperature: 650°C
Time: 3 hours

The degree of crystallinity of the obtained specimens was measured in the same manner as in Example 1. The result is described below.
Degree of crystallinity: 75%

### Evaluation

Calcium elution rates were measured for Example 2 and Comparative Example 1. In addition, silver elution amounts were measured, and antimicrobial tests were performed for Example 1 and Comparative Example 1. Each measurement method and results are described below.

### Calcium Elution Rate

Specimens were immersed in Tris buffer at 37°C and pH 7.3. The calcium concentration (mg/L) in the Tris buffer was measured using ICP mass spectrometry at each time shown in Table 1. The results are shown in Table 1.

**[Table 1]**

| | Specimen | | Calcium concentration (mg/L) | | | | | | |
|---|---|---|---|---|---|---|---|---|---|
| | Degree of crystallinity (%) | Processing conditions | 0.0 d | 0.2 d | 0.3 d | 1.0 d | 3.0 d | 7.0 d | 14.0 d |
| Example 2 | 100 | Hydrothermal treatment | 0 | 0.2 | 0.4 | 1.2 | 2.6 | 4.5 | 5.9 |
| Comparative Example 1 | 75 | Vacuum thermal treatment | 0 | 3.9 | 5.6 | 8.1 | 10.0 | 10.4 | 11.2 |

As can be seen from Table 1, Example 2 had lower calcium concentrations in the Tris buffer after 14 days than Comparative Example 1. Thus, it can be seen that, in Example 2, the dissolution rate was slower than that of Comparative Example 1, and the period until the coating film disappears was long.

### Silver Elution Rate

The specimens were immersed in bovine serum at 37°C. The silver concentration (mg/L) in the bovine serum was measured using ICP mass spectrometry at each time shown in Table 2. The evaluation results are shown in Table 2.

**[Table 2]**

| | Specimen | | Silver concentration (mg/L) | | | | |
|---|---|---|---|---|---|---|---|
| | Degree of crystallinity (%) | Processing conditions | 0 h | 4 h | 12 h | 24 h | 72 h |
| Example 1 | 91 | Hydrothermal treatment | 0 | 31 | 39 | 52 | 106 |
| Comparative Example 1 | 75 | Vacuum thermal treatment | 0 | 27 | 57 | 86 | 143 |

As can be seen from Table 2, Example 1 showed lower silver concentrations in the bovine serum after 72 days than Comparative Example 1. Thus, it can be seen that compared with Comparative Example 1, in Example 1, the coating film more easily maintains its silver content, and the antimicrobial properties can be maintained over a long period of time.

### Antimicrobial Test

The antimicrobial activity value (R-value) was measured by a coating film adhesion method in accordance with JIS Z 2801. The test conditions are as follows.
Bacterial species: MRSA/UOEH6
Medium: Non-activated bovine serum

The measurement results are as follows.
Antimicrobial activity value
Example 1: 0.5
Comparative Example 1: 3.6

A microscopic observation by SEM was performed for Examples 1 and 2 and Comparative Example 1. A scanning electron microscope (SEM: SN - 3400N, manufactured by Hitachi, Ltd.) was used. As a result, in Examples 1 and 2 and Comparative Example 1, a plurality of flat molten particles were observed on the surface of the silver HA thermally sprayed coating film. In Examples 1 and 2, a plurality of HA needle-like crystals were observed on the entire surface of the plurality of molten particles, and a state in which the plurality of HA needle-like crystals are located in the gap at the interface between adjacent ones of the molten particles of calcium phosphate was observed. but, in Comparative Example 1, such a state was not observed.

In Comparative Example 1, the inside of the coating film was mainly formed of fine crystals of HA. In contrast, in Examples 1 to 2, HA crystals had grown in a dendritic form inside the coating film (molten particles of calcium phosphate), and dendritic crystals were contained inside the coating film.

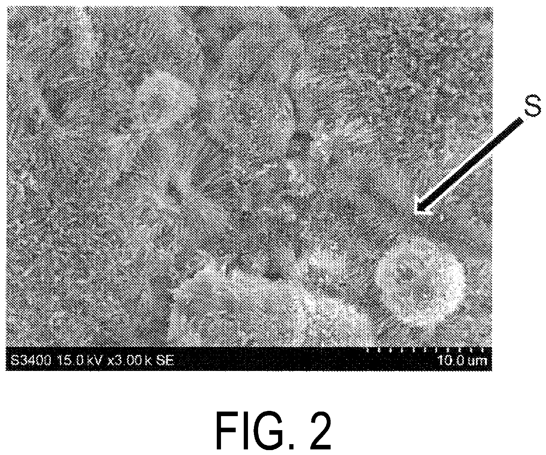

A SEM photograph of the surface of the specimen of Comparative Example 1 is shown in FIG. 2. The scale is illustrated in the lower right of FIG. 2. Note that one mark indicates 1 µm, and ten marks indicate 10 µm. As described above, a plurality of flat molten particles were observed on the surface, a plurality of HA needle-like crystals were observed on the entire surface of the plurality of molten particles, and a state in which the plurality of HA needle-like crystals are located in the gap S at the interface between adjacent ones of the molten particles was observed.

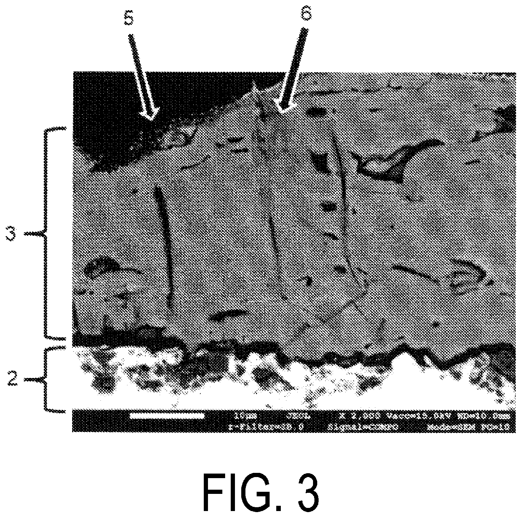

A SEM photograph of the cross section near the surface including the coating film of the specimen of Example 2 is shown in FIG. 3. The scale is illustrated in the lower left of FIG. 3, with a white bar length of 10 µm. In a cross-sectional view, the plurality of needle-like crystals 5 on the surface of the coating film 3 can be confirmed. It can be confirmed that the dendritic crystals 6 are located between the particles inside the coating film 3.

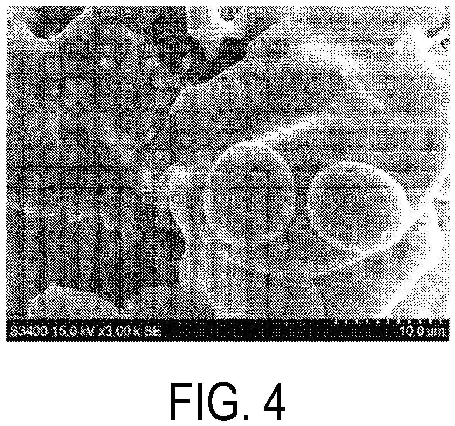

A SEM photograph of the surface of the specimen of Comparative Example 1 is shown in FIG. 4. The scale is illustrated in the lower right of FIG. 4. Note that one mark indicates 1 µm, and ten marks indicate 10 µm. As described above, a plurality of flat molten particles were observed, but needle-like crystals were not observed. The specimens of Comparative Example 1 was further subjected to a hydration treatment described in patent 6192014 (immersed from 10 to 60 minutes in water from 60 to 100°C). Although the needle-like crystals were observed on the surface, the state in which the plurality of HA needle-like crystals are located in the gap at the interface between adjacent ones of the molten particles was not observed.

### Reference Signs List

- 1: Biocompatible implant
- 2: Base
- 3: Calcium phosphate coating film
- 4: Calcium phosphate particle
- 41: Calcium phosphate first particle
- 42: Calcium phosphate second particle
- S: Gap at interface between calcium phosphate particles
- 5: Needle-like crystal
- 6: Dendritic crystal

## Claims

1. A biocompatible implant comprising:
a base; and
a calcium phosphate coating film located on a surface of the base and including silver, wherein
the calcium phosphate coating film comprises a plurality of calcium phosphate particles located on a surface thereof, and a plurality of needle-like crystals located on a surface of the plurality of calcium phosphate particles, and
the plurality of needle-like crystals are located in a gap at an interface between adjacent calcium phosphate particles among the plurality of calcium phosphate particles.

2. The biocompatible implant according to claim 1, wherein
the degree of crystallinity of the calcium phosphate coating film is greater than 90%.

3. The biocompatible implant of claim 1 or claim 2, wherein
the calcium phosphate coating film includes dendritic crystals located therein.

4. The biocompatible implant according to any one of claims 1 to 3, wherein
the calcium phosphate coating film is a calcium phosphate thermally sprayed coating film.

5. The biocompatible implant according to any one of claims 1 to 4, wherein
the silver is metal silver or a silver compound and is dispersed and distributed in the calcium phosphate coating film as irregularly shaped particles each being 1 nm or greater.

6. The biocompatible implant according to any one of claims 1 to 5, wherein
a thickness of the calcium phosphate coating film is from 5 to 300 µm.

7. The biocompatible implant according to any one of claims 1 to 6, wherein
the calcium phosphate coating film contains any one or more of hydroxyapatite, α-tricalcium phosphate, β-tricalcium phosphate or tetracalcium phosphate.

8. The biocompatible implant according to any one of claims 1 to 7, wherein
a particle size of each of the plurality of calcium phosphate particles is from 0.5 to 100 µm.

9. The biocompatible implant according to any one of claims 1 to 8, wherein
each of the plurality of needle-like crystals has a major axis from 0.1 to 50 µm and a minor axis from 0.001 to 10 µm.

10. The biocompatible implant according to any one of claims 1 to 9, wherein
the base includes any one of a metal, a ceramics or a plastic.

11. The biocompatible implant according to any one of claims 1 to 10, wherein
the biocompatible implant is any one of an artificial bone, an inner fixing tool, a prosthetic joint, a dental implant or a spinal implant.

12. A method of manufacturing a biocompatible implant comprising:
thermal spraying a calcium phosphate raw material containing silver onto a base for a biocompatible implant to form a coating film on a surface of the base; and
forming a plurality of needle-like crystals on the surface of the coating film by subjecting the coating film to hydrothermal treatment in which the coating film is heated to a temperature greater than 100°C in a pressure vessel containing water.

13. The method of manufacturing the biocompatible implant according to claim 12, wherein
processing conditions of the hydrothermal treatment are temperature: from 130 to 155°C, and time: from 11 to 25 hours.
